# EUROPEAN PATENT SPECIFICATION

(11) **EP 2 647 648 B1**
(45) Date of publication and mention of the grant of the patent: **09.08.2017**
(21) Application number: 11845938.7
(22) Date of filing: 01.12.2011
(51) Int. Cl.: C08L 1/28, C08B 11/08, A61K 9/20, A61K 47/38, C08B 11/20, C09D 101/28, A61K 9/16, A61K 9/28, A61K 9/50

(54) **Solid dosage form containing a low viscosity HYDROXYALKYL CELLULOSE**
Feste Darreichungsform enthaltend eine niedrigviskose HYDROXYALKYLCELLULOSE
Forme galénique solide contenant de l'HYDROXYALKYLCELLULOSE a faible viscosité

(30) Priority: 03.12.2010 JP 2010270172
(43) Date of publication of application: 09.10.2013
(73) Proprietor: Nippon Soda Co., Ltd., Tokyo 100-8165 (JP)
(72) Inventor: ABE Satoru, Joetsu-shi Niigata 949-2392 (JP); KATO Takashi, Joetsu-shi Niigata 949-2392 (JP)
(74) Representative: Wills, Andrew Jonathan
(86) International application number: PCT/JP2011/077778
(87) International publication number: WO 2012/074042

(56) References cited:
- WO-A1-2005/027876
- WO-A1-2009/061815
- WO-A1-2009/157564
- WO-A2-2009/061821
- JP-A- 2000 296 322
- "Excipients Nisso HPC Hydroxypropyl Cellulose", Nippon Soda Co. Ltd. , 2011, XP002728138, Retrieved from the Internet: URL:http://www.nissoexcipients.com/PDF/TDS -01.pdf [retrieved on 2014-08-04]

## Description

### TECHNICAL FIELD

The present invention relates to a hydroxyalkyl cellulose that is suitable for producing an orally-disintegrating agent, a gastro-soluble solid formulation, an entero-soluble solid formulation, a slow-release solid formulation, a bitterness-suppressing solid formulation, and the like.

Priority is claimed on Japanese Patent Application No. 2010-270172, filed December 3, 2010.

### BACKGROUND ART

A method of producing an orally-disintegrating agent or the like, which is for masking bitterness of a dry syrup or facilitating taking of the syrup, by using coating particles obtained by coating core particles having a particle size of 100 µm or less with drug particles has been developed. Moreover, use of core particles having a particle size of 100 µm or less as a drug delivery system such as an oral agent or an injection is drawing attention.

When core particles having a particle size of 100 µm or less are covered with a drug (layering) and granulated, a binder is used. However, the core particles having a particle size of 100 µm or less are extremely easily aggregated due to binding of interparticle bridges that are formed by a coating agent containing the binder. Consequently, the amount of drug particles attached to the core particles (drug layering efficiency) is reduced. As one of the means for inhibiting aggregation of the core particles, use of a coating agent having a weak binding force is considered. However, if the coating agent having a weak binding force is used, the binding force between the core particles and the drug particles is weakened. Therefore, the drug particles covering the core particles are easily detached, and the drug layering efficiency is reduced.

Non-Patent Literatures 1 and 2 disclose that the use of a coating agent containing hydroxypropyl cellulose, which has a viscosity of 2.0 mPa-s to 2.9 mPa-s in a 2%-concentration aqueous solution at 20°C, as a binder suppresses the aggregation of core particles to some extent and increases the drug layering efficiency.

Meanwhile, as tablet production methods, direct tableting, granulation and compression, and the like are known. It is known that in these production methods, hydroxypropyl cellulose is used as a binder so as to improve compression moldability.

WO 2009/061821 A2 describes cellulose ether compositions for film-forming coating applications, including a coating composition which contains an aqueous solution of either a very low viscosity cellulose ether or a low-hydroxypropyl cellulose ether.

Excipients Datasheet TDS-01 from Nisso HPC details the properties of a number of grades of hydroxypropyl cellulose.

WO 2009/061815 A1 describes methods for producing a very low viscosity cellulose ether having little or no discoloration and cellulose ether products resulting therefrom.

WO 2005/027876 A1 describes stable pharmaceutical compositions of acid-labile benzimidazole derivative using increased amounts of low-viscosity hydroxypropyl cellulose, and processes for the preparation of these compositions.

WO 2009/157564 A1 describes a cellulose composition comprising 55 to 75 mass% of crystalline cellulose [A] which has an average particle diameter of 80 to 150 µm, a loose bulk density of 0.30 to 0.50 g/cm³ and an angle of repose of 40° or less; 17.5 to 37.5 mass% of a water-soluble cellulose derivative [B] which has an average particle diameter of 80 µm or less and can be prepared into a 2 mass% aqueous solution having a viscosity of 10 mPa·s or less at 20°C; 0.01 to 0.5 mass% of a fluidising agent [C]; and 5 to 20 mass% of an excipient [D] having an average particle diameter of 150 µm or less and a loose bulk density of 0.60 to 1.00 g/cm³.

### PRIOR ART LITERATURE

### Non-Patent Literature

[NPL I] Ichikawa H., Fukumori Y., Adeyeye C. M. Int. J. pharm., 156, 39-48 (1997)
[NPL 2] Ichikawa H., Aoyagi K., Fukumori Y., AAPS Journal, 7(S2), 301 (2005)

### DISCLOSURE OF INVENTION

### Problems to be Solved by the Invention

However, when core particles having a particle size of 100 µm or less are covered with a drug (layering) and granulated, the coating agent containing Hydroxypropyl cellulose, which has a viscosity of 2.0 mPa·s to 2.9 mPa·s in a 2%-concentration aqueous solution at 20°C, as a binder neither completely inhibits the aggregation of the core particles nor perfectly attaches the drug to every single core particle. Moreover, a disintegration time of a tablet, which is formed using the hydroxypropyl cellulose having a viscosity of 2.0 mPa·s to 2.9 mPa·s in a 2%-concentration aqueous solution at 20°C, is prolonged in some cases. The disintegration time can be shortened by reducing the amount of binder. However, in this case, the hardness of the tablet is reduced, and this makes it difficult to obtain rapid disintegration properties with a high degree of hardness.

Therefore, the present invention aims to provide a binder that further reduces the aggregation rate of core particles, further increases the drug layering efficiency, and is suitable for obtaining tablets having excellent tablet hardness and disintegration properties.

### Means for Solving the Problems

In order to solve the above problems, the present inventors conducted a thorough examination. As a result, they obtained a hydroxyalkyl cellulose that has a viscosity of 1.10 mPa·s to 1.95 mPa·s in a 2%-concentration aqueous solution at 20°C. Moreover, by using a coating agent containing the hydroxyalkyl cellulose as a binder, they attempted to cover core particles, particularly, core particles having a volume average primary particle size of 100 µm or less with drug particles. As a result, they found that the aggregation rate of the core particles is further reduced, and the drug layering efficiency is further increased, compared to a case of using the coating agent containing hydroxypropyl cellulose, which has a viscosity of 2.0 mPa·s to 2.9 mPa·s in a 2%-concentration aqueous solution at 20°C, as a binder. They also found that a solid formulation, which is formed by compression molding by using hydroxyalkyl cellulose that has a viscosity of 1.10 mPa·s to 1.95 mPa·s in a 2%-concentration aqueous solution at 20°C as a binder, has a more sufficient tablet strength and is superior in disintegration time, compared to a solid formulation which is formed by compression molding by using hydroxypropyl cellulose that has a viscosity of 2.0 mPa·s to 2.9 mPa·s in a 2%-concentration aqueous solution at 20°C as a binder. By further repeating examination based on these findings, the present inventors completed the present invention.

That is, the present invention includes the following embodiments.
(1) A solid formulation which is in a compression-molded form of coated particles, each coated particle comprising:
   a core particle; and
   a coating layer covering the core particle
   wherein the coating layer contains a hydroxyalkyl cellulose that has a viscosity of 1.10 mPa·s to 1.95 mPa·s in a 2%-concentration aqueous solution at 20°C, and has a content of hydroxyalkyl group in a range of 40% by weight to 80% by weight.
(2) The solid formulation according to (1), wherein the solid formulation is a tablet.
(3) The solid formulation according to (2), wherein the content of the hydroxyalkyl cellulose in the solid formulation is 1% by weight to 30% by weight.
(4) The solid formulation according to (1), wherein a volume average primary particle size of the core particles is 5 µm to 1000 µm.
(5) The solid formulation according to (1), wherein the content of the hydroxyalkyl cellulose in the coating layer is 1 % by weight to 50% by weight.
(6) The solid formulation according to (1), wherein the coating layer further contains drug particles.
(7) The solid formulation according to (1), wherein a volume average primary particle size of the drug particles is smaller than a volume average primary particle size of the core particles.

### Effects of the Invention

The hydroxyalkyl cellulose of the present invention can make it possible for drug particles to almost completely cover and be attached to every single core particle (that is, the hydroxyalkyl cellulose can increase a drug layering efficiency), particularly, core particles having an average primary particle size of 100 µm or less, while almost completely inhibiting the aggregation of the core particles.

Moreover, the hydroxyalkyl cellulose of the present invention can make it possible to provide solid formulations such as tablets having sufficient tablet strength and excellent disintegration time.

### BRIEF DESCRIPTION OF THE DRAWINGS

FIG. 1 is a schematic view showing an example of a jet layer-type coating device.
FIG. 2 is a view showing a yield of coated particles of Example 4 and Comparative examples 1 to 3 at various spray pressure values.
FIG. 3 is a view showing an aggregation rate of coated particles of Example 4 and Comparative examples 1 to 3 at various spray pressure values.
FIG. 4 is a view showing a drug layering efficiency of coated particles of Example 4 and Comparative examples 1 to 3 at various spray pressure values.
FIG. 5 is a view showing a diameter of a sprayed liquid drop of Example 4 and Comparative examples 1 to 3 at various spray pressure values.

### DETAILED DESCRIPTION

### (Hydroxyalkyl cellulose)

The hydroxyalkyl cellulose of the present invention has a viscosity of 1.10 mPa·s to 1.95 mPa·s and preferably has a viscosity of 1.5 mPa·s to 1.9 mPa·s in a 2%-concentration aqueous solution at 20°C. If the viscosity of the hydroxyalkyl cellulose is too low, the binding force thereof tends to be reduced. On the other hand, if the viscosity of the hydroxyalkyl cellulose is too high, core particles are aggregated seriously, whereby the drug layering efficiency is reduced.

The hydroxyalkyl cellulose is obtained by, for example, preparing alkali cellulose by allowing sodium hydroxide to act on cellulose as a raw material, and then causing a substitution reaction between the alkali cellulose and alkylene oxide. After the substitution reaction, an acid such as acetic acid or hydrochloric acid can be added to the reaction liquid to neutralize the sodium hydroxide, and then the resultant can be purified. By the substitution reaction, a portion or all of -OH groups in a glucose ring unit of cellulose are substituted with a -O-(R-O)ₘ-H group. Herein, R represents a divalent alkyl group. m represents the number of repeated R-O in parentheses and is a natural number equal to or greater than 1.

The content of a hydroxyalkyl group (-(R-O)ₘ-H) in the hydroxyalkyl cellulose is preferably in a range of 40% by weight to 80% by weight, and more preferably in a range of 53% by weight to 78% by weight. The content of the hydroxyalkyl group can be measured by the method according to USP 24 (United States Pharmacopeia) and the like.

Examples of the alkylene oxide used in the substitution reaction include ethylene oxide, propylene oxide, and the like. Among these, propylene oxide is preferably used in the present invention. If the substitution reaction is caused using propylene oxide, hydroxypropyl cellulose is obtained.

The method for producing the hydroxyalkyl cellulose of the present invention is not particularly limited. Examples of the method include a method for producing the hydroxyalkyl cellulose by using low-molecular-weight cellulose as a raw material; a method for producing the hydroxyalkyl cellulose by dissolving hydroxyalkyl cellulose obtained by a general production method in water so as to precipitate the hydroxyalkyl cellulose in the form of a gel, separating and collecting the supernatant liquid, and removing water; and the like.

### (Solid formulation)

The solid formulation of the present invention contains the hydroxyalkyl cellulose of the present invention. In the present invention, the solid formulation refers to granules, tablets (including sugar-coated tablets, film-coated tablets, sublingual tablets, and orally-disintegrating tablets), and the like, and preferably refers to tablets (including sugar-coated tablets, film-coated tablets, sublingual tablets, and orally-disintegrating tablets). Generally, the solid formulation contains active ingredients (for example, in the case of medications or agrochemicals, drugs and active ingredients for health foods) and optionally further contains additives such as an excipient, a binder, a disintegrator, a lubricant, a release-retarding agent, a base, a colorant, a pH adjuster, a pH buffer, a surfactant, a stabilizer, an acidulant, a flavoring, a fluidizer, a refreshing agent, a sweetener, an umami flavor compouent, a sweetness enhancer.

The hydroxyalkyl cellulose of the present invention is contained in the solid formulation, mainly as an ingredient functioning as a binder or a base.

The content of the hydroxyalkyl cellulose used in the solid formulation of the present invention is not particularly limited, but the content is preferably 1% by weight to 30% by weight and more preferably 3% by weight to 20% by weight in the solid formulation.

Examples of drugs as active ingredients of medications include an analgesic, an antipyretic analgesic, a headache cure, a cough remedy, an expectorant, a sedative, an antispasmodic, an antihistaminic, an antiallergic agent, an antiplasmin agent, a bronchodilator, an agent for treating asthma, an agent for treating diabetes, an agent for treating liver disease, an agent for treating ulcers, an agent for treating gastritis, a stomachic digestant, a gastrointestinal motility activator, an agent for treating hypertension, an agent for treating angina, an antihypertensive, an agent for treating hypotension, an agent for treating hyperlipidemia, a hormone agent, an antibiotic, an antiviral agent, a sulfa drug, an anti-inflammatory agent, an agent for psychoneurosis, an ocular hypotensive agent, an antiemetic, an antidiarrheic, an agent for treating gout, an agent for treating arrhythmia, a vasoconstrictor, a digestant, an agent introducing (inducing) sleep or hypnosis, a sympatholytic, an agent for treating anemia, an antiepileptic, an anti-vertigo agent, an agent for treating balance disorder, an agent for treating tuberculosis, an agent for treating avitaminosis, an agent for treating dementia, an agent for treating urinary incontinence, an anti-motion sickness agent, an oral germicide, helminthics, a vitamin supplement, amino acids, minerals, and the like. More specifically, the examples include drugs for central nervous system (acetaminophene, aspirin, indomethacin, ibuprofen, naproxen, declofenac sodium, meclofenoxate hydrochloride, chlorpromazine, tolmetin sodium, milnacipran hydrochloride, Phenobarbital, and the like), drugs for peripheral nervous system (etomidoline, tolperisone hydrochloride, pipethanate ethylbromide, methyl benactyzium bromide, flopropione, and the like), hemostats (carbazochrome sodium sulfate, protamine sulfate, and the like), drugs for circulatory organs (aminophylline, etilefrine hydrochloride, diltiazem hydrochloride, digitoxin, captopril, and the like), drugs for respiratory organs (ephedrine hydrochloride, chlorprenaline hydrochloride, oxeladin citrate, cloperastine, sodium chromoglycate, and the like), drugs for digestive organs (berberine chloride, loperamide hydrochloride, cimetidine, ranitidine hydrochloride, famotidine, and the like), coronary vasodilators (nifedipine, nicardipine, verapamil, and the like), vitamin supplements (ascorbic acid, thiamine hydrochloride, calcium pantothenate, rivoflavin butyrate, and the like), metabolic formulations (camostat mesilate, mizoribine, lysozyme chloride, and the like), antiallergic drugs (cyproheptadine hydrochloride, diphenhydramine hydrochloride, alimemazine tartrate, splatast tosilate, diphenhydramine maleate, and the like), chemotherapeutic agents (acyclovir, enoxacin, ofloxacin, pipemidic acid trihydrate, levofloxacin, and the like), antibiotics (erythromycin, cefcapene pivoxil hydrochloride, cefteram pivoxil, cefpodoxime proxetil, cefaclor, cefalexin, clarithromycin, rokitamycin, and the like), and the like.

Examples of drugs as active ingredients of agrochemicals include a bactericide, an antiviral agent, a germicide, a miticide, a pesticide, a nematocide, a rodenticide, a herbicide, a plant growth regulator, a fertilizer, a phytotoxicity reducer, and the like.

Among the above compounds as active ingredients of medications or agrochemicals, the compounds having a salt-forming moiety also include physiologically or pharmaceutically acceptable salts thereof (particularly, medically or agrochemically acceptable salts, and the like) and the like.

The active ingredients of health foods refer to ingredients that are mixed in to enhance health. Examples thereof include powder of aojiru, aglycone, *Agaricus subrufescens*, *A*shwagandha, astaxanthin, acerola, amino acids (valine, leucine, isoleucine, lysine, methionine, phenylalanine, threonine, tryptophan, histidine, cystine, tyrosine, arginine, alanine, aspartic acid, seaweed powder, glutamine, glutamic acid, glycine, proline, serine, and the like), alginic acid, gingko leaf extract, sardine peptides, turmeric, uronic acid, Echinacea, *Eleutherococcus senticosus*, oligosaccharide, oleic acid, nucleoprotein, bonito peptides, catechin, potassium, calcium, carotinoid, garcinia cambogia, L-carnitine, chitosan, conjugated linoleic acid, *Aloe arborescens*, gymnema sylvestre extract, citric acid, Orthosiphon stamineus, glyceride, glycerol, glucagon, glutamine, glucosamine, L-glutamine, chlorella, cranberry extract, cat's claw, germanium, enzymes, Korean ginseng extract, coenzyme Q10, collagen, collagen peptides, Coleus forskolin, chondroitin, psyllium husk powder, hawthorn extract, saponin, lipids, L-cystine, Japanese basil extract, citrimax, fatty acid, plant sterol, seed extract, spirulina, squalene, white willow extract, ceramide, selenium, ST John's wort extract, soy bean isoflavone, soybean saponin, soybean peptides, soybean lecithin, monosaccharide, proteins, chest tree extract, iron, copper, docosahexaenoic acid, tocotrienol, nattokinase, natto bacillus culture extract, niacin sodium, nicotinic acid, disaccharide, lactobacillus, garlic, Serenoa repens, germinated rice, Job's tears extract, herb extract, Valerian extract, pantothenic acid, hyaluronic acid, biotin, chromium picolinate, vitamin A, A2, B1, B2, B6, B12, C, D, E, and K, hydroxytyrosol, bifidobacteria, beer yeast, fructooligosaccharide, flavonoid, butcher's bloom extract, black cohosh, blueberry, prune extract, proanthocyanidin, proteins, propolis, bromelain, probiotics, phosphatidylcholine, phosphatidylserine, β-carotene, peptides, safflower extract, maitake mushroom extract, maca extract, magnesium, milk thistle, manganese, mitochondria, minerals, mucopolysaccharide, melatonin, phellinus linteus, melilot extract powder, molybdenum, vegetable powder, folic acid, lactose, picoline, linoleic acid, lipoic acid, phosphorus, lutein, lecithin, rosmarinic acid, royal jelly, DHA, EPA, and the like.

Examples of the release-retarding agent include sodium alginate, a carboxyvinyl polymer; acrylic acid-based polymers such as an aminoalkyl methacrylate copolymer RS [Eudragit RS (trade name), manufactured by Rohm Pharma Company] and ethyl acrylate-methyl methacrylate copolymer suspension [Eudragit NE (trade name), manufactured by Rohm Pharma Company], and the like.

Examples of the excipient include starches such as corn starch, potato starch, wheat starch, rice starch, partly pregelatinized starch, pregetatinized starch, and porous starch; saccharides and sugar alcohols such as lactose, fructose, glucose, D-mannitol, sorbitol, trehalose, and erythritol; anhydrous calcium phosphate, crystalline cellulose, precipitated calcium carbonate, calcium silicate, and the like.

Examples of the disintegrator include carboxymethylcellulose, carboxymethylcellulose calcium, carboxymethyl starch sodium, croscarmellose sodium, crospovidone, hydroxypropyl starch, and the like.

Examples of the binder include crystalline cellulose, polyvinylpyrrolidone, gum Arabic, and the like.

Examples of the lubricant include magnesium stearate, calcium stearate, talc, sucrose fatty acid ester, sodium stearyl fumarate, and the like.

Examples of the colorant include food colorings such as Food Yellow No. 5, Food Red No. 2, and Food Blue No. 2, food lake dyes, iron sesquioxide, and the like.

Examples of the pH adjuster include a citric acid salt, a phosphoric acid salt, a carbonic acid salt, a tartaric acid salt, a fumaric acid salt, an acetic acid salt, amino-acid salt, and the like.

Examples of the pH buffer include a citric acid-sodium citrate buffer and the like.

Examples of the surfactant include sodium lauryl sulfate, polysorbate, polyoxyethylene polyoxypropylene glycol, and the like.

Examples of the stabilizer include tocopherol, tetrasodium edetate, nicotinic acid amide, cyclodextrins, and the like.

Examples of the acidulant include ascorbic acid, citric acid, tartaric acid, malic acid, and the like.

Examples of the flavoring include menthol, peppermint oil, lemon oil, vanillin, and the like.

Examples of the fluidizer include soft silicic anhydride, hydrated silicon dioxide, and the like.

Examples of the refreshing agent include terpene-based compounds (monoterpene alcohol and the like) such as camphor and borneol, refined oil, essence, or powder containing the terpene-based compounds; refined oil, essence, or powder of peppermint, spearmint, cool mint, and the like; the refined oil or essence that is adsorbed onto a powdery carrier (for example, dextrin), agents obtained by mixing the refined oil or essence with an excipient (gum Arabic or the like) and a liquid base (water, alcohol, or the like) and granulating the resultant, and the like.

Examples of the sweetener include non-carbohydrate-based sweeteners, sugar alcohol, saccharides, and the like. As the non-carbohydrate-based sweetener, any of synthetic and natural sweeteners can be used.

Examples of the tasty ingredient include amino acid-based tasty ingredients (amino acids or a salt thereof, for example, glutamic acid, sodium glutamate, potassium glutamate, glutamic acid hydrochloride, sodium guanylate, inosinic acid, sodium inosinate, arginine-glutamic acid salt, aspartic acid, sodium aspartate, glycine, and alanine), peptide-based tasty ingredients (dipeptide such as L-glutamyl-Lglutamate and L-glutamyl-L-serine; tripeptide such as tri-L-glutamate and L-glutamyl-L-glycyl-L-serine; and the like), carboxylic acid-based tasty ingredients (carboxylic acid salts such as sodium succinate, and the like), and the like.

In addition, the solid formulation may contain a sweetness enhancer (or an achar) having a salty taste (saline taste). Examples of the sweetness enhancer include sodium chloride, potassium chloride, phosphoric acid salts (potassium monohydrogen phosphate, sodium hydrogen phosphate, and the like), and the like. The sweetness enhancer (or achar) is a neutral salt, for example, a salt dissociated as a sodium ion and/or a chlorine ion (chloride ion), in many cases.

The production method of a solid formulation is not particularly limited. Examples of the method include a method in which an excipient or a disintegrator is added to and mixed with a main drug, the resultant is kneaded with fine particles of the hydroxyalkyl cellulose according to the present invention so as to be granulated by using a granulating machine or the like, followed by drying and granulation, a lubricant such as magnesium stearate is mixed with the resultant, and the mixture is subjected to tableting (wet granulation and tableting or dry granulation and tableting); a method in which a main drug, an excipient, and fine particles of the hydroxyalkyl cellulose according to the present invention are mixed together, a lubricant is mixed with the resultant, and the mixture is subjected to tableting (dry direct tableting); and the like.

### (Coated particles)

The hydroxyalkyl cellulose of the present invention can be used for producing coated particles. The coated particles include core particles and a coating layer covering the core particles. The coating layer is a layer containing the hydroxyalkyl cellulose of the present invention.

### <Core particles>

The core particles used in the coated particles may be particles including only the active ingredient exemplified above (for example, in the case of medications or agrochemicals, drugs and active ingredients for health foods), particles including a mixture of a carrier and an active ingredient, particles in which an active ingredient covers the surface of a carrier, or particles including a carrier that does not contain an active ingredient at all. The core particles can be used without particular limitation, as long as they do not cause shape collapse during operation.

The upper limit of the volume average primary particle size of the core particles is preferably 1000 µm, more preferably 500 µm, and particularly preferably 100 µm. In addition, the lower limit of the volume average primary particle size of the core particles is generally 5 µm and preferably 10 µm. The volume average primary particle size is a value of particle size D₅₀ accounting for a cumulative 50% in particle size distribution that is obtained by measuring the particle size by using a laser diffraction-type particle size distribution analyzer (for example, LDSA-2400; manufactured by Tonichi Computer Applications Co., Ltd.) under the condition of a pneumatic pressure of 3.5 kgf/cm² and a focal distance of 100 mm. Moreover, the particle shape can be observed with a scanning electron microscope (for example, JSM-7330; manufactured by JEOL Ltd.).

As the core particles, for example, a pill, granules, powder, a single crystal of a drug, an aggregate of drug powder, particles of lactose, hydroxyapatite, calcium carbonate particles; crystalline cellulose granules that are commercially available as coating core particles in the field of drug production, spherical sucrose granules, spherical mannitol granules, and the like can be used.

The core particles may be controlled release formulations such as a rapid-release formulation and a sustained-release formulation (slow-release formulation). The core particles may contain commonly used additives. Examples of the additives include an excipient, a disintegrator, a binder, a lubricant, a colorant, a pH adjuster, a pH buffer, a surfactant, a release-retarding agent, a stabilizer, an acidulant, a flavoring, a fluidizer, a refreshing agent, a sweetener, a tasty ingredient, a sweetness enhancer, and the like. Examples of these additives include those described above, and these additives are used in the amount commonly used in the field of drug production.

Examples of other ingredients that can be contained in the core particle also include an antioxidant or an oxidation inhibitor, a dispersant, a suspension, a solubilizing aid, a thickener (water-soluble polymers such as a carboxyvinyl polymer, polyvinyl alcohol, and gelatin; cellulose ethers such as carboxymethylcellulose), an antiseptic or a preservative (parabens such as methylparaben and butylparaben), a germicide or a bactericide (benzoic acids such as sodium benzoate), an antistatic agent, a taste-correcting agent or a masking agent, a smell-correcting agent, a defoamer, a tonicity agent, a soothing agent, and the like. One kind of these additives may be used alone, or two or more kinds thereof may be used in combination. The production process of the core particles is not particularly limited and can employ a general granulation method.

### <Coating layer>

The content of the hydroxyalkyl cellulose in the coating layer is not particularly limited. The content is preferably 1% by weight to 50% by weight and more preferably 3% by weight to 30% by weight in the coating layer.

The coating layer preferably further contains drug particles.

Examples of the drug particles include particles of the active ingredients exemplified above.

The size of the drug particles is not particularly limited and generally smaller than the size of the core particles. The volume average primary particle size of the drug particles is preferably 0.1 µm to 100 µm, more preferably 0.5 µm to 50 µm, and particularly preferably 1 µm to 10 µm.

The coating layer may further contain a substance (binding force enhancer) that has a function of enhancing the binding force between the core particles and the drug particles. Examples of the substance include organic fatty acids (lauric acid, palmitic acid, myristic acid, stearic acid, and the like), ester derivatives of organic fatty acids, higher alcohols (cetyl alcohol, stearyl alcohol, and the like), glycerin fatty acid esters (glyceryl monostearate and the like), polyethylene glycols (macrogol 6000 and the like), wax-like substances such as natural wax (carnauba wax, rice wax, and the like), and the like. Among these, at least one kind selected from the group consisting of a polyalkylene glycol, a polyalkylene glycol higher fatty acid ester, a higher fatty acid, a higher alcohol, a higher alcohol ester, and natural wax is preferable, and polyethylene glycol is particularly preferable. As a binding force enhancer, a hydrophilic enhancer is preferable. Moreover, the melting point of the binding force enhancer is preferably 40°C to 70°C and particularly preferably 50°C to 65°C. The content of the binding force enhancer in the coating layer is not particularly limited. The content is preferably 0.1% by weight to 20% by weight and more preferably 0.5% by weight to 15% by weight in the coating layer.

The coating layer may contain another coating base. The volume average particle size of the coating base is preferably 0.1 µm to 100 µm and more preferably 0.1 µm to 50 µm.

Examples of another coating base include polymer bases, inorganic granules, and the like. In addition, the additives exemplified above as additives that can be contained in the core particles can be used as another coating base.

Examples of the polymer bases include synthetic or natural polymers. Specific examples thereof include acrylic polymers, in vivo-degradable polymers, polyvinyl-based polymers, and the like.

Examples of the acrylic polymers include an aminoalkyl methacrylate copolymer E, a methacrylic acid-methyl methacrylate copolymer, and the like. Examples of the in vivo degradable polymers include homopolymers or copolymers including L-lactic acid, D-lactic acid, DL-lactic acid, glycolic acid, ε-caprolactone, and N-methylpyrrolidone, or a mixture of these polymers, polycaprolactam, chitin, chitosan, and the like. Examples of the polyvinyl-based polymers include polyvinylacetal diethylaminoacetate, a PVA copolymer, and the like.

As the polymer base, an elution-controlling base such as an entero-soluble coating base, a gastro-soluble coating base, a water-insoluble coating base, a slow-release coating base, or a water-soluble coating base can be preferably used. Among these, a water-insoluble coating base is preferably exemplified. One kind of these polymer bases can be used alone, or two or more kinds thereof can be used in combination. For example, a combination of a water-insoluble coating base and an entero-soluble coating base, and a combination of a water-insoluble coating base and a water-soluble coating base are preferably exemplified.

As an entero-soluble coating base, a polymer that is practically insoluble in an acidic liquid and is soluble in an alkaline liquid can be used. Examples thereof include a methacrylic acid copolymer LD (Eudragit L30D55; manufactured by Evonik Industries), a methacrylic acid copolymer L (Eudragit L100; manufactured by Evonik Industries), a methacrylic acid copolymer S (Eudragit S100; manufactured by Evonik Industries), hydroxypropyl methylcellulose phthalate (HPMCP), hydroxypropyl methylcellulose acetate succinate (AQOAT), carboxymethyl ethylcellulose (CMEC), cellulose acetate phthalate (CAP), polyvinyl acetate phthalate (PVAP), cellulose acetate trimellitate (CAT), Aquateric (aqueous dispersion of CAP), zein, and the like.

As the water-insoluble coating base, the substances that are practically not dissolved in water but are dissolved or evenly dispersed in organic solvents such as methanol, ethanol, propanol, isopropanol, and acetone can be used. For example, ethyl cellulose; a water-insoluble natural resin such as shellac; a water-insoluble acrylic polymer such as an aminoalkyl methacrylate copolymer RS (Eudragit RS; manufactured by Evonik Industries) and a methacrylic acid copolymer RSPO (Eudragit RSOP; manufactured by Evonik Industries), and the like are preferable. Among these, a water-insoluble acrylic polymer is preferable.

Examples of the water-soluble coating base include methyl cellulose, sodium carboxymethyl cellulose, polyvinylpyrrolidone, and the like.

Examples of the inorganic granules include talc, sodium chloride, sodium citrate, soft silicic anhydride (silica), precipitated calcium carbonate, magnesium stearate, calcium stearate, titanium oxide, and the like. Among these, silica is preferable. If the inorganic granules are mixed in, the fluidity of the solid formulation is improved.

The coated particles of the present invention are obtained by covering the core particles with the coating layer by using a known coating method.

Examples of the coating method include the methods disclosed in publications such as "Granulation Handbook" (edited by The Association of Powder Process Industry and Engineering, JAPAN, Ohmsha, Ltd.), "Formulation of Preparation for Oral Administration" (edited by Prof. Mitsuru HASHIDA, Graduate School and Faculty of Pharmaceutical Sciences, Kyoto University, Yakugyo Jiho, Inc), "Particle Design Engineering" (edited by The Society of Powder Technology, JAPAN, Sangyo-Tosho Publishing Co., Ltd.), and "Particle Design and Preparation Technology" (edited by Yoshiaki Kawashima, president of The Society of Powder Technology, Japan/Division of Particulate Design and Preparations, Yakugyo Jiho, Inc). In the present invention, a fluidized coating method is preferable, and a jet-layer coating method is particularly preferable.

Devices for performing fluidized coating include a fluidized bed-type coating device, ajet-layer type coating device, and a fluid-rolling type coating device. Among these, a jet-layer type coating device is particularly preferable.

The bottom portion of the jet-layer type coating device is configured with a screen through which air flow blown up from the bottom can pass. Due to the air flow, particles supplied onto the screen are caused to float and be fluidized inside the device. To the flowing particle layer, a coating agent is sprayed. Examples of the spray method include a method of spraying the coating agent from the top (top spray), a method of spraying the coating agent from the bottom (bottom spray), a method of spraying the coating agent from the side (tangential spray), and the like. Among these, the top spray method is generally used. Drying is performed by using air flow blown up from the portion below the screen.

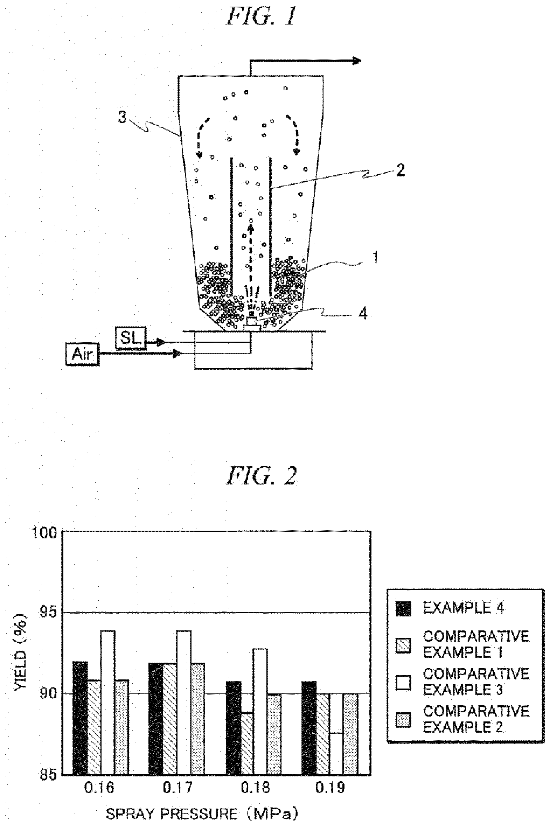

The jet-layer type coating device includes a cylindrical internal tube (guiding tube) inside the device, and is configured to blow up the screen only from the portion below the guiding tube. Since the air flow is blown up from the bottom at a high speed inside the guiding tube, the particles float up, whereby a jet-layer of the particles is formed. The particles thrown up fall toward the outside of the guiding tube, slip down to the bottom, and then are thrown up again by the air flow. A spray nozzle is positioned at the center of the screen, and generally employs a method of spraying a coating agent to the top (bottom spray). A spray pressure is preferably 0.05 MPa to 0.5 MPa, more preferably 0.1 MPa to 0.3 MPa, and particularly preferably 0.15 MPa to 0.2 MPa. A diameter of a sprayed liquid drop (D₅₀) is preferably 1 µm to 30 µm, more preferably 3 µm to 20 µm, and particularly preferably 8 µm to 13 µm. Drying is performed by the air flow blown up from the portion below the screen. FIG. 1 is a schematic view showing an example of a jet-layer type coating device.

The fluid-rolling type coating device is a device that is obtained by installing a rolling disk in the bottom portion of the jet-layer type coating device instead of the screen, and fluidities particles by using the air flow blown up from the periphery of the rolling disk.

After coating, the solid formulation as the coated particles can be optionally subjected to another coating process such as film coating, sugar coating, thin-layer sugar coating, sugarless sugar coating, or sugarless thin-layer sugar coating. Moreover, when coating granules, fine granules, or drug particles are obtained, these can be made into tablets by being subjected to compression molding together with other excipients. In addition, the coated particles can be filled in capsules so as to be made into capsules. Furthermore, the particles can be taken as they are by being packed individually as granules or fine granules. Alternatively, the particles can be made into a formulation dissolved upon use, a tablet rapidly disintegrated in the oral cavity, a slow-release formulation, a film sheet-type formulation, a gummy formulation, or a jelly formulation. Examples

Next, the present invention will be described in more detail with reference to examples, but the present invention is not limited to the examples.

### Reference Example 1

Hydroxypropyl cellulose that has a viscosity of 2.2 mPa·s in a 2%-concentration aqueous solution at 20°C was dissolved in water at a concentration of 15%. This aqueous solution was heated up to about 62°C, and as a result, a portion of the hydroxypropyl cellulose was precipitated in the form of gel. The supernatant liquid was separated and collected and diluted with water, thereby obtaining an aqueous solution containing 2%-concentration hydroxypropyl cellulose. The viscosity of the hydroxypropyl cellulose at 20°C was 1.70 mPa·s. Water was removed from the supernatant liquid, thereby obtaining hydroxypropyl cellulose.

### Reference Example 2

Hydroxypropyl cellulose having a viscosity of 1.84 mPa·s in a 2%-concentration aqueous solution at 20°C was obtained in the same manner as in Example 1, except that the heating temperature was changed to 58°C.

### Reference Example 3

Hydroxypropyl cellulose having a viscosity of 1.93 mPa·s in a 2%-concentration aqueous solution at 20°C was obtained in the same manner as in Example 1, except that the heating temperature was changed to 55°C.

Coated particles were evaluated in the following manner.

### (Aggregation rate)

The particle size distribution of CCSS coated particles was measured using an electromagnetic vibration sieve (manufactured by TSUTSUI SCIENTIFIC INSTRUMENTS CO., LTD., the amount of sample added: 1 g). The particles of each fraction were observed with a digital microscope, and as a result, particles in a fraction not lower than an inflection point in a logarithmic normal probability plot of cumulative % of undersize particles were considered as aggregates. Therefore, a proportion of the amount of particles classified to be not lower than an inflection point in the total amount of particles was taken as an aggregation rate.

### (Drug layering efficiency)

10 mg of CCSS coated particles were put in a measuring flask having a volume of 100 ml, purified water was poured into the flask, and the CCSS was dissolved and extracted. Thereafter, purified water was added to the flask so as to prepare 100 ml of a solution. This solution was filtered through a 0.1 µm membrane filter. The absorbance of the filtrate at a measurement wavelength of 363 nm was measured using an ultraviolet/visible light absorptiometer (UV-150-02, manufactured by Shimadzu Corporation). Based on a calibration curve created in advance, the CCSS content was calculated from the measured absorbance. A drug layering efficiency was obtained by multiplying the total mass of the coated particles obtained in the coating operation by the CCSS content calculated above, and dividing the result value by the total mass of the CCSS supplied to the coating operation.

### (Diameter of spray liquid drop)

A spray liquid was sprayed at a predetermined spray pressure in an open environment at room temperature. By using a laser-scattering type particle size distribution analyzer (LDSA-2400A, manufactured by Tonichi Computer Applications Co., Ltd.), the sprayed liquid drops were measured in a position 40 mm distant from the tip of a spray gun. The measurement was performed three times, and the average was calculated.

### Reference Example 4

1.5 parts by weight of hydroxypropyl cellulose, which was obtained in Example 1 and had a viscosity of 1.70 mPa·s in a 2%-concentration aqueous solution at 20°C, was dissolved in 80 parts by weight of distilled water. In this solution, 4 parts by weight of carbazochrome sodium sulfonate (CCSS, average particle size 4 µm, manufactured by Sanwa Chemical Co., Ltd.) was dispersed, thereby obtaining a spray liquid (SL).

50 parts by weight of lactose having a primary particle size of 63 µm to 75 µm was put in a jet-layer coating device (a guiding tube length of 170 mm, a vent blower frequency of 12 Hz) shown in FIG. 1, and air (Air) of 65°C and the spray liquid (SL) were sprayed at rates of 0.15 m³/min and 1.4 ml/min respectively from the bottom of the device at a predetermined spray pressure. A vent temperature was 26°C to 30°C.

By the above coating operation, CCSS coated particles were obtained. The evaluation results thereof are shown in FIGS. 2 to 5.

### Comparative example 1

CCSS coated particles were obtained in the same manner as in Example 4, except that Hydroxypropyl cellulose having a viscosity of 2.68 mPa·s in a 2%-concentration aqueous solution at 20°C was used instead of hydroxypropyl cellulose having a viscosity of 1.70 mPa·s in a 2%-concentration aqueous solution at 20°C. The evaluation results thereof are shown in FIGS. 2 to 5.

### Comparative example 2

CCSS coated particles were obtained in the same manner as in Example 4, except that low-viscosity polyvinylpyrrolidone (PVP-10, manufactured by Sigma-Aldrich Co.) having a weight-average molecular weight of about 10000 was used instead of hydroxypropyl cellulose having a viscosity of 1.70 mPa·s in a 2%-concentration aqueous solution at 20°C. The evaluation results thereof are shown in FIGS. 2 to 5.

### Comparative example 3

CCSS coated particles were obtained in the same manner as in Example 4, except that high-viscosity polyvinylpyrrolidone (PVP-40T, manufactured by Sigma-Aldrich Co.) having a weight-average molecular weight of about 40000 was used instead of hydroxypropyl cellulose having a viscosity of 1.70 mPa·s in an aqueous solution of 20°C containing the hydroxypropyl cellulose at a concentration of 2%. The evaluation results thereof are shown in FIGS. 2 to 5.

FIG. 2 is a view showing the yield of coated particles. In any of Example 4 and Comparative examples 1 to 3, the yield was 90% or higher in general. The product loss is considered to result from the leakage of the particles outside the chamber and the pass of the particles through the bag filter. In Comparative example 3, product loss occurred since the particles were attached to the guiding tube at a spray pressure of 0.19 MPa. It is considered that this is because the core particles are blown up excessively due to the jet stream caused by the high-pressure sprayer, and the amount of the core particles flowing inside the guiding tube becomes insufficient, whereby the sprayed liquid drops that are supposed to come into contact with the core particles become too attached to the guiding tube.

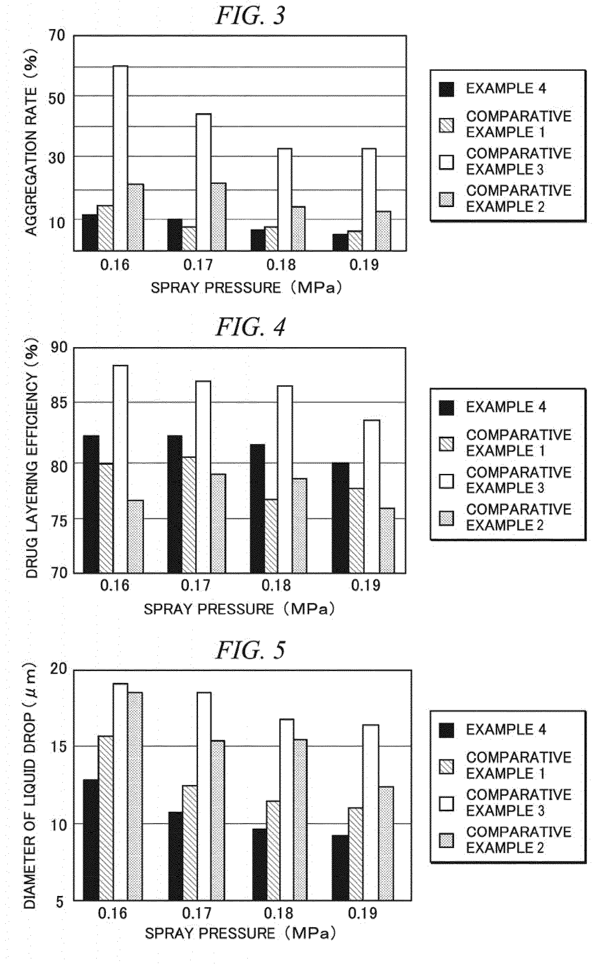

FIG. 3 is a view showing the aggregation rate of particles. As the spray pressure increased, the aggregation rate tended to be reduced. At any spray pressure, the coated particles of Example 4 are aggregated less compared to the coated particles of Comparative examples 1 to 3. FIG. 5 is a view showing the diameter of sprayed liquid drops. From the drawing, it is confirmed that the higher the spray pressure, the smaller the diameter of liquid drops. Comparing FIG. 3 to FIG. 5, it is found that the diameter of liquid drops is correlated with the aggregation rate. That is, as the diameter of liquid drops decreases, the aggregation rate tends to decrease. At a constant concentration, the larger the diameter of liquid drops, the more the amount of a binder contained in the liquid drop increases. It is considered that for this reason, the particles are more easily crosslinkes to each other, and aggregation is promoted.

FIG. 4 is a view showing the drug layering efficiency. In the comparison between Comparative example 2 and Comparative example 3, the drug layering efficiency was higher in a case of using high-viscosity polyvinylpyrrolidone than in a case of using low-viscosity polyvinylpyrrolidone.

However, in the comparison between Example 4 and Comparative example 1, the drug layering efficiency was higher in a case of using low-viscosity Hydroxypropyl cellulose according to the present invention than in a case of using high-viscosity hydroxypropyl cellulose.

### Example 5

The Hydroxypropyl cellulose that was obtained in Example 1 and had a viscosity of 1.70 mPa·s in a 2%-concentration aqueous solution at 20°C was dissolved in distilled water, thereby obtaining an aqueous solution containing HCP at a concentration of 8%.

210 parts by weight of lactose (manufactured by DMV International, Pharmatose, 200M) and 90 parts by weight of corn starch (NIHON SHOKUHIN KAKO CO., LTD., Corn Starch W) were put in a rolling flow type granulation device (manufactured by FREUND, SPIR-A-FLOW MINI model). The mixture was granulated at a granulation temperature of 30.3°C to 40.0°C, while 112.5 parts by weight of the aqueous HPC solution obtained above was being sprayed at a flow rate of 5 ml/min, a spray pressure of 1 kg/cm², a rotation frequency of a rotor of 300 rpm, and a temperature of supplied air of 70.5°C to 72.0°C. In this manner, particles having an average particle size D₅₀ of 89 µm were obtained.

The particles were subjected to compression molding at a tableting pressure of 10 KN and a rotation frequency of tableting of 10 rpm, by using a rotary tableting machine (manufactured by KIKUSUI SEISAKUSHO, LTD., VELA5 0312SS 2MZ). In this manner, lens-shaped tablets having a diameter of 8 mm and a tablet weight of 200 mg were obtained. The evaluation results of the hardness and disintegration time of the obtained tablets are shown in Table 1.

### Comparative example 4

Particles having an average particle size D₅₀ of 107 µm were obtained in the same manner as in Example 5, except that hydroxypropyl cellulose having a viscosity of 2.69 mPa·s in a 2%-concentration aqueous solution at 20°C was used instead of hydroxypropyl cellulose having a viscosity of 1.70 mPa·s in a 2%-concentration aqueous solution at 20°C. By using the particles, tablets were obtained in the same manner as in Example 5. The evaluation results of the hardness and disintegration time of the obtained tablets are shown in Table 1.

**[Table 1]**

| | Tablet hardness (kg) | Disintegration time (min) |
|---|---|---|
| Example 5 | 10.1 | 4.3 |
| Comparative example 4 | 9.5 | 5.4 |

The tablet hardness was measured using a tablet hardness tester (manufactured by ERWEKA GmbH, TBH28 model). The disintegration time was evaluated by a method (test fluid: water, 37°C) based on the disintegration test of The Japan Pharmacopoeia, by using a disintegration tester (manufactured by Toyama Sangyo Co., Ltd., NT-2 model).

From the results of Example 4 and Comparative examples 1 to 3, it is understood that the coated particles obtained using the hydroxyalkyl cellulose of then present invention have a low aggregation rate and a high drug layering efficiency.

In addition, from the results of Example 5 and Comparative example 4, it is understood that the tablets obtained using the hydroxyalkyl cellulose of the present invention have a high degree of hardness and can shorten the disintegration time. Reference Signs List

1: core particle
2: guiding tube
3: jet-fluidized layer
4: nozzle
SL: spray liquid
Air: air

## Claims

1. A solid formulation which is in a compression-molded form of coated particles, each coated particle comprising:
a core particle; and
a coating layer covering the core particle
wherein the coating layer contains a hydroxyalkyl cellulose that has a viscosity of 1.10 mPa·s to 1.95 mPa·s in a 2%-concentration aqueous solution at 20°C, and has a content of hydroxyalkyl group in a range of 40% by weight to 80% by weight.

2. The solid formulation according to Claim 1, wherein
the solid formulation is a tablet.

3. The solid formulation according to Claim 1, wherein
the content of the hydroxyalkyl cellulose in the solid formulation is 1% by weight to 30% by weight.

4. The solid formulation according to Claim 1, wherein
a volume average primary particle size of the core particles is 5 µm to 1000 µm.

5. The solid formulation according to Claim 1, wherein
the content of the hydroxyalkyl cellulose in the coating layer is 1% by weight to 50% by weight.

6. The solid formulation according to Claim 1, wherein
the coating layer further contains drug particles.

7. The solid formulation according to Claim 6, wherein
a volume average primary particle size of the drug particles is smaller than a volume average primary particle size of the core particles.

## Patentansprüche

1. Feste Formulierung, die in einer formgepressten Form von beschichteten Teilchen vorliegt, wobei jedes beschichtete Teilchen Folgendes umfasst:
ein Kernteilchen; und
eine Beschichtungsschicht, die das Kernteilchen umhüllt;
wobei die Beschichtungsschicht eine Hydroxyalkylcellulose mit einer Viskosität bei 20 °C von 1,10 mPa·s bis 1,95 mPa·s in einer wässrigen Lösung mit einer Konzentration von 2 % enthält und einen Hydroxyalkylgruppengehalt im Bereich von 40 Gew.-% bis 80 Gew.-% aufweist.

2. Feste Formulierung nach Anspruch 1, wobei die feste Formulierung eine Tablette ist.

3. Feste Formulierung nach Anspruch 1, wobei der Gehalt der Hydroxyalkylcellulose in der festen Formulierung 1 Gew.-% bis 30 Gew.-% beträgt.

4. Feste Formulierung nach Anspruch 1, wobei die volumsmittlere Primärteilchengröße der Kernteilchen 5 µm bis 1000 µm beträgt.

5. Feste Formulierung nach Anspruch 1, wobei der Gehalt der Hydroxyalkylcellulose in der Beschichtungsschicht 1 Gew.-% bis 50 Gew.-% beträgt.

6. Feste Formulierung nach Anspruch 1, wobei die Beschichtungsschicht weiters Arzneimittelteilchen enthält.

7. Feste Formulierung nach Anspruch 6, wobei die volumsmittlere Primärteilchengröße der Arzneimittelteilchen kleiner ist als die volumsmittlere Primärteilchengröße der Kernteilchen.

## Revendications

1. Formulation solide qui est sous une forme moulée par compression de particules revêtues, chacune particule revêtue comprenant :
une particule centrale ; et
une couche de revêtement recouvrant la particule centrale
dans laquelle la couche de revêtement contient une hydroxyalkylcellulose qui a une viscosité comprise entre 1,10 mPa·s et 1,95 mPa·s dans une solution aqueuse à concentration de 2 % à 20 °C, et a une teneur en groupe hydroxyalkyle dans une plage de 40 % en poids à 80 % en poids.

2. Formulation solide selon la revendication 1, dans laquelle
la formulation solide est un comprimé.

3. Formulation solide selon la revendication 1, dans laquelle
la teneur en hydroxyalkylcellulose dans la formulation solide est de 1 % en poids à 30 % en poids.

4. Formulation solide selon la revendication 1, dans laquelle
une taille de particule primaire moyenne en volume des particules centrales est de 5 µm à 1 000 µm.

5. Formulation solide selon la revendication 1, dans laquelle
la teneur en hydroxyalkylcellulose dans la couche de revêtement est de 1 % en poids à 50 % en poids.

6. Formulation solide selon la revendication 1, dans laquelle
la couche de revêtement contient en outre des particules de médicament.

7. Formulation solide selon la revendication 6, dans laquelle
une taille de particule primaire moyenne en volume des particules de médicament est inférieure à une taille de particule primaire moyenne en volume des particules centrales.
